# EUROPÄISCHE PATENTSCHRIFT

(11) **EP 3 506 952 B1**
(45) Veröffentlichungstag und Bekanntmachung des Hinweises auf die Patenterteilung: **29.12.2021**
(21) Anmeldenummer: 17768994.0
(22) Anmeldetag: 31.08.2017
(51) Int. Cl.: A61L 2/00, A61L 2/08

(54) **INAKTIVIERUNG VON PATHOGENEN IN BIOLOGISCHEN MEDIEN**
INACTIVATION OF PATHOGENS IN BIOLOGICAL MEDIA
INACTIVATION D'AGENTS PATHOGÈNES DANS DES MILIEUX BIOLOGIQUES

(30) Priorität: 01.09.2016 DE 102016216573
(43) Veröffentlichungstag der Anmeldung: 10.07.2019
(73) Patentinhaber: Fraunhofer Gesellschaft zur Förderung der angewandten Forschung e.V., 80686 München (DE)
(72) Erfinder: THOMA, Martin, 70771 Leinfelden-Echterdingen (DE); FISCHER, Klaus, 37359 Großbarthoff (DE); PORTILLO, Javier, 01277 Dresden (DE)
(74) Vertreter: Schrell, Andreas
(86) Internationale Anmeldenummer: PCT/EP2017/071879
(87) Internationale Veröffentlichungsnummer: WO 2018/041953

(56) Entgegenhaltungen:
- WO-A1-2015/011265
- DE-A1-102013 012 455
- GB-A- 2 424 877
- GB-A- 2 424 877
- US-A1- 2009 081 340
- US-A1- 2009 081 340

## Beschreibung

Die Erfindung betrifft die Verarbeitung flüssiger biologischer Medien, besonders von Kulturmedien, Zell- oder Virussuspensionen, gegebenenfalls enthaltend aktive Pathogene, mit dem Ziel der Inaktivierung dieser Pathogene und/oder der Modifikation von Inhaltsstoffen in diesen biologischen Medien durch ionisierende Betastrahlung.

Bekanntermaßen können pathogene Substanzen, darunter Toxine oder Erreger wie Viren, Viruspartikel, Bakterien oder andere Organismen, durch Exposition mit thermischer oder ionisierender Strahlung vornehmlich UV-, Röntgen- oder GammaStrahlung sowie Betastrahlung inaktiviert werden. Dabei werden die Pathogene derart modifiziert, dass ihre pathogene Wirkung auf einen tierischen oder menschlichen Mechanismus oder eine Zell- oder Gewebekultur minimiert oder vollständig aufgehoben wird. Solche thermische oder ionisierende Strahlung, zum Beispiel mit nicht-thermischen Elektronen (Betastrahlung) verändert auf molekularer Ebene die strukturelle Integrität einzelner oder mehrerer struktur- oder funktionsbestimmender Komponenten dieser Pathogene und bewirken so deren Inaktivierung. Problematisch dabei ist, dass, einer Dosis-Wirkungskorrelation folgend, eine zu geringe Strahlendosis zu einer unvollständigen oder unzureichenden Inaktivierung der Pathogene führt, eine zu hohe Strahlendosis aber unerwünschte Strukturänderungen und Modifikationen in weiteren Komponenten der biologischen Medium verursachen kann. Dies ist insbesondere problematisch bei der Herstellung von Impfstoffen aus Suspensionen aktiver Pathogene, insbesondere aus Virussuspensionen. Werden beispielsweise Virussuspensionen mit niederenergetischer Betastrahlung bestrahlt, um die Viren zu deaktivieren, wie dies in der DE 10 2013 012 455 A1 beschrieben ist, führt zu geringe Strahlendosis zu einer unerwünschten, unvollständigen Inaktivierung der Viren, zu hohe Strahlendosis hingegen zu einer Zerstörung oder teilweisen Denaturierung der Viren oder viralen Antigenstrukturen und damit zu einer Beeinträchtigung der immunogenen Wirkung des herzustellenden Impfstoffes. Weder bei zu niedriger, noch bei zu hoher Strahlendosis wäre die Virensuspension dann als Impfstoff einsetzbar.

Eine Bestrahlung mit thermischer oder ionisierender Strahlung kann aber auch zur gezielten Modifikation, das heißt Umwandlung, Mutagenese, Stimulation, Transduktion von Zellen oder Geweben in der Zellforschung und bei der Zell- und Gewebeherstellung eingesetzt werden, besonders auch bei der die Fragmentierung der zellulären DNA, um eine Zellproliferation zu unterbinden. Dabei ist eine Kontrolle der richtigen Dosis von hoher Bedeutung, insbesondere wenn Dosis-Wirkungs-Abhängigkeiten solcher strahlungsbedingter Modifikationen und Wirkungen an Zellen oder Geweben erst wissenschaftlich bestimmt werden sollen.

Im Rahmen der Automatisierung biotechnologischer Anlagen, insbesondere Anlagen zur Herstellung von Impfstoffen, aber auch Anlagen der Zell- und Gewebekultur, sollen Vorrichtungen, Verfahren und Mittel geschaffen werden, welche eine insbesondere kontinuierliche Verarbeitung von biologischen Medien, vor allem pathogenen Suspensionen, Suspensionen von Zellen oder Geweben, sterilen Medien etc. ermöglichen. Dabei ist es wünschenswert, Verfahren und Mittel zur dosiskontrollierten Bestrahlung solcher biologischer Medien bereitzustellen, die insbesondere vollautomatisch und kontinuierlich betreibbar sind und den laufenden Stofffluss solcher Anlagen "eingeschaltet" werden können. Gleichzeitig soll die systembedingt erforderliche Reinigbarkeit, Austauschbarkeit oder Sterilisierbarkeit solcher Mittel möglich sein. Ebenfalls soll der Personenschutz (Infektion) und Produktschutz (Kontamination) gewährleistet sein.

Die GB 2 424 877 A beschreibt eine Vorrichtung zur Erzeugung eines kontinuierlichen Flüssigkeitsfilms in einer Anordnung zur kontinuierlichen dosiskontrollierten Bestrahlung. Die US 2009/081340 A1 beschreibt eine Vorrichtung zur kontinuierlichen UV- oder Elektronenbestrahlung einer Flüssigkeit zu deren Desinfektion.

Der Erfindung lag das technische Problem zugrunde, Verfahren und Mittel zur automatisierbaren kontinuierlichen Bestrahlung von flüssigen biologischen Medien bereitzustellen, welche geeignet sind, insbesondere innerhalb einer Automatenstraße für die Herstellung oder Bearbeitung solcher Medien einen kontinuierlichen Flüssigkeitsstrom das biologische Medium mit einer kontrollierbaren Strahlendosis zu beaufschlagen. Dadurch soll eine verbesserte, das heißt insbesondere dosiskontrollierte Strahlenexposition, vor allem zum Zwecke der zuverlässigen Inaktivierung von Pathogenen oder zur gezielten strahlungskorrelierten Modifikation von biologischen Medien erreicht werden. Gleichzeitig soll ein integrales Modul bereitgestellt werden, welches innerhalb der Automatenstraßen autonom betreibbar ist und austauschbar und leicht reinigbar ist sowie Personenschutz und Produktschutz gewährleistet sind.

Das technische Problem wird gelöst durch eine neuartige Vorrichtung zur kontinuierlichen Vergleichmäßigung eines flüssigen Mediums zur Exposition des vergleichmäßigten Mediums mit Betastrahlung. Gemäß der Erfindung wird dazu ein Modul bereitgestellt, welches zur Erzeugung eines kontinuierlichen Flüssigkeitsfilms aus kontinuierlich zugeführter Flüssigkeit geeignet ist und die Bestrahlung dieses Flüssigkeitsfilms, welcher eine vorbestimmbare Dicke, das heißt Oberflächen/Volumen-Verhältnis aufweist, ermöglicht. Das Modul ist erfindungsgemäß in Form einer austauschbaren integralen Kassette ausgeführt. Die Kassette ist, besonders separat von der Anordnung, worin sie zur wiederkehrenden Verwendung zur kontinuierlichen dosiskontrollierten Bestrahlung eingesetzt werden kann, sterilisierbar. Die Kassette besteht gemäß der Erfindung aus einem Modulgehäuse mit einer Wanne zur Aufnahme der kontinuierlich zugeführten Flüssigkeit und mit einer Zylinderwalze, welche in diese Wanne, und insbesondere in die darin aufgenommene zugeführte Flüssigkeit eintaucht, und dort entlang einer Achse rotierbar ist. Weiter besitzt das Modul zumindest einen Zulaufkanal zur Zufuhr der Flüssigkeit in die Wanne. Es ist mindestens eine Abstreiflippe vorgesehen, welche in dichtem Kontakt mit der Walzenoberfläche der Zylinderwalze steht, und zwar auf der in Rotationsrichtung der Walze ablaufenden Seite der Walze, zum Abstreifen des bei Rotation der Walze auf der umlaufenden Walzenoberfläche aus der in der Wanne aufgenommenen Flüssigkeit gebildeten Flüssigkeitsfilms. Ein Ablaufkanal zur Aufnahme und kontinuierlichen Ableitung von mit dieser Abstreiflippe von der Walzenoberfläche abgestreifter Flüssigkeit ist ebenfalls vorgesehen.

Erfindungsgemäß ist an der Wanne ein Überlaufkanal vorgesehen zum Ablauf von überschüssig zugeführter Flüssigkeit aus der Wanne, um den Flüssigkeitspegel in der Wanne festzulegen und konstant zu halten.

Erfindungsgemäß besteht die Kassette weiter aus einem Gehäusedeckel, welcher das Modulgehäuse dicht verschließt, um die austauschbare Kassette zu bilden. Der Gehäusedeckel weist mindestens ein strahlungsdurchlässiges Fenster (Strahlungsfenster) in Form eines Metallfensters auf.

Ein bevorzugtes Element einer ersten Ausgestaltung des erfindungsgemäßen Modulgehäuses der Kassette ist ein spezielles spaltbildendes Element, welches auf der bei Rotation auflaufenden Seite der Walze angeordnet ist, dort, wo die zugeführte Flüssigkeit aus der Wanne auf die Walzenoberfläche aufläuft. Das spaltbildende Element ist zu der Walzenoberfläche so beanstandet, dass dort ein Kapillarspalt gebildet wird. Dieser Kapillarspalt erstreckt sich zumindest bis oberhalb des Flüssigkeitspegels in der Wanne. Dieses spaltbildende Element dient erfindungsgemäß der Erzeugung und Vergleichmäßigung des auf der Walzenoberfläche gebildeten Flüssigkeitsfilms.

Die Erfinder fanden überraschend, dass durch das Zusammenwirken der erfindungsgemäßen Elemente ein sehr kontinuierlicher Flüssigkeitsfilm aus einer kontinuierlich zugeführten Flüssigkeit erzeugt werden kann, welcher im laufenden Betrieb eine konstante Dicke, besonders ein konstantes Oberflächen/VolumenVerhältnis aufweist. Der durch den erfindungsgemäß vorgesehenen Überlaufkanal an der Wanne des Modulgehäuses konstant gehaltene Flüssigkeitspegel in der Wanne kann, besonders in Verbindung mit dem bevorzugten spezifischen spaltbildenden Element, welches mit der Walzenoberfläche einen Kapillarspalt bildet, der sich bevorzugt bis oberhalb dieses Flüssigkeitspegels erstreckt, einen überraschend gleichmäßigen kontinuierlichen Flüssigkeitsfilm auf der Walze bewirken. In einer dieser bevorzugten Ausgestaltungen kann spezifisch über die Breite des Kapillarspalts und/oder über die Höhe des sich oberhalb des Flüssigkeitspegels der Wanne erstreckenden Anteils des Kapillarspalts die Dicke des Flüssigkeitsfilms in gewissen Grenzen eingestellt werden.

Art und Qualität der Flüssigkeit, deren Viskosität, sowie Art und Qualität der benetzten Oberflächen, insbesondere der Oberfläche der Walze, sowie die Umlaufgeschwindigkeit der Walze spielen ebenfalls eine Rolle bei der Ausbildung eines geeigneten Flüssigkeitsfilms.

In einer alternativen Ausgestaltung des erfindungsgemäßen Modulgehäuses der Kassette ist auf ein solches spezielles spaltbildendes Element verzichtet. Besonders weil in bestimmten Varianten auch ohne ein solches speziell ausgeformtes und angeordnetes spaltbildendes Element ausreichend zuverlässig ein kontinuierlicher Flüssigkeitsfilm auf der rotierenden Walze erzeugt werden kann.

In einer bevorzugten Ausgestaltung ist der mindestens eine Überlaufkanal an der Wanne in der Höhe oder Eintauchtiefe veränderlich, wodurch der Flüssigkeit Pegel in der Wanne eingestellt oder vorbestimmt werden kann.

In einer bevorzugten Ausgestaltung ist zusätzlich zu dem Überlaufkanal mindestens ein weiteres Mittel zu Festlegung, das heißt Konstanthaltung des Flüssigkeitspegels in der Wanne des Moduls vorgesehen. Die Konstanthaltung des Flüssigkeitspegels in der Wanne kann bevorzugt dadurch verbessert werden, dass an dem Überlaufkanal ein Blasendetektor oder Durchflussmesser angebracht ist, welcher die Flussrate und/oder auftretende Gasblasen detektiert. Art und Qualität des Flusses am Überlaufkanal gibt indirekt Aufschluss über den Flüssigkeitspegel in der Wanne des Moduls. Das Sensorsignal kann verwendet werden, um die Zulaufrate an dem Zulaufkanal entsprechend zu steuern. Eine Blasendetektion dient vor allem der "Funktionskontrolle". Ein kontinuierlicher Strom, ohne Blasen am Überlaufkanal ließe darauf schließen, dass die Flüssigkeit im Zulauf Gefahr läuft in das Reservoir für inaktivierte Flüssigkeit zu laufen, was verhindert werden soll. Darüber hinaus kann über die Blasendetektion bei Inbetriebnahme kontrolliert werden, ob die Kassette sachgemäß angeschlossen und verschlaucht wurde.

Die Druckmessung dient vor allem der Dichtigkeitskontrolle des geschlossenen Systems vor oder während des Betriebs. Das Modulinnere wird mit einem geringen Über- oder Unterdruck beaufschlagt. Sollte das Modul undicht sein, äußert sich dies durch eine Druckänderung.

In einer weiteren alternativen Ausgestaltung ist die Flüssigkeitszuführung auf die Walze als so genanntes Kammerrakel ausgeführt. Die Kammer wird bevorzugt durch Dichtlippen an der Walze abgedichtet. Alternativ bildet die Kammer mit der Walze einen dichtenden Kapillarspalt. Der Flüssigkeitsauftrag auf die Walze erfolgt fluss- und/oder druckgesteuert.

In allen Ausführungen ist bevorzugt vorgesehen, dass die Abstreiflippe an der ablaufenden Seite der Walze entgegen die Ablaufrichtung der rotierenden Walze gerichtet ist. Sie funktioniert wie ein entgegen der Rotationsrichtung gerichteter Schaber. Bevorzugt wird die Abstreiferlippe durch die Rotation auf die Walzenoberfläche gedrückt und liegt dort spaltfrei und bevorzugt über ihre gesamte Breite an. Von der Walzenoberfläche abgestreifte Flüssigkeit läuft über die Abstreiferlippe bevorzugt in eine an der Verankerung des Abstreifers mit dem Modulgehäuse angeordnete Ablaufrinne oder Ablaufkanal und kann so gesammelt und aus dem Modulgehäuse abgeführt werden.

In ein der dazu alternativen bevorzugten Variante ist die Abstreiflippe an der ablaufenden Seite der Walze so angeordnet, dass sie in Ablaufrichtung der rotierenden Walze gerichtet ist. Sie bildet mit der Oberfläche der Walze eine querverlaufende Rinne, worin sich die abgestreifte Flüssigkeit sammeln kann. Von dort kann sie passiv überlaufen und an einer an der Verankerung des Abstreifers angeordneten Ablaufrinne gesammelt und aus dem Modulgehäuse abgeführt werden. Alternativ und bevorzugt ist der Ablaufkanal dort aber als mindestens ein in die zwischen Abstreiflippe und der ablaufenden Seite der Walze gebildete Rinne ragendes Rohr ausgebildet. Die Drainage der abgestreiften Flüssigkeit aus der Rinne über dieses Rohr kann vorzugsweise aktiv erfolgen, besonders bevorzugt durch Absaugen mittels Vakuumpumpe, vorzugsweise eine Schlauchpumpe, oder alternativ durch Austreiben über Überdruck; dies setzt allerdings besonders ein Reservoir mit Druckausgleich voraus.

In einer weiteren alternativen Ausgestaltung ist die Flüssigkeitsabführung von der Walzenoberfläche über eine doppelte Abstreiflippe vermittelt. Zumindest die nachlaufende (untere) Abstreiflippe liegt an der Walze an und ist in Ablaufrichtung der rotierenden Walze gerichtet. Beide parallel verlaufenden Abstreiflippen bilden zusammen mit der Walzenoberfläche eine Kammer in der Weise eines Kammerrakels. Die Kammer bildet den Ablaufkanal.

In einer bevorzugten Ausgestaltung ist auf der auflaufenden Seite der Walze, entsprechend stromabwärts des gebildeten Kapillarspalts, zusätzlich mindestens ein Abstreifer angeordnet, der zu der Walzenoberfläche beabstandet ist. Der Abstreifer dient der zusätzlichen Vergleichmäßigung des Flüssigkeitsfilms und der Einstellung der Schichthöhe für besonders viskose Medien. Der Abstreifer ist in bevorzugter Variante dieser Ausführung seinem Abstand zu der Walzenoberfläche einstellbar oder durch entsprechend unterschiedlich dimensionierte Abstreifer in dem Modul austauschbar, um die Wirkung auf die Vergleichmäßigung des Flüssigkeitsfilms und auf die Dicke des Flüssigkeitsfilms und je nach Anforderung zu steuern. Die Abstreifkante des Abstreifers weist dabei in einer besonderen Variante dieser Ausführung eine Kreisbogen- oder Ellipsenform auf, um den Flüssigkeitsfilm zwischen dem zentralen Abschnitt und den peripheren Abschnitten der Walze zu vergleichmäßigen.

Bevorzugte Materialien der Walzenoberfläche sind ausgewählt aus Elektronen beziehungsweise Wärmestrahlen reflektierenden, besonders bevorzugt ausgewählt aus Metallen der VIII. Nebengruppe, besonders bevorzugt aber aus den Metallen Platin (Pt), Gold (Au), Chrom (Cr), Nickel (Ni) und Eisen (Fe) sowie legierten Eisenstählen, besonders Chrom-Nickel-Stahl und anderen Edelstählen. Besonders bevorzugt ist eine Gold (Au)- Beschichtung, alternativ bevorzugt ist eine Platin (Pt)-Beschichtung. Alternativ oder zusätzlich ist die Walzenoberfläche mittels an sich bekannter chemischer oder Plasmaverfahren hydrophilisiert, um dort die Ausbildung eines geschlossenen Flüssigkeitsfilms zu verbessern. Alternativ oder zusätzlich ist eine Strukturierung der Oberfläche der Walze vorgesehen.

In einer bevorzugten Ausgestaltung ist die Walze an ihre Oberfläche durch geeignete Maßnahmen temperiert, das heißt insbesondere gekühlt, um eine strahlungsbedingte Aufheizung zu kompensieren, insbesondere um unerwünschte nachteilige Strahlungswirkungen zu verhindern. In einer alternativen Variante ist die Walzenoberfläche aufheizbar, um insbesondere die Strahlungswirkung in Verbindung mit der thermischen Einwirkung zu verstärken oder gezielt auf thermisch sensitive Strukturen in dem flüssigen Medium zu richten. Dazu ist besonders ein zusätzlicher Kreislauf zum Durchleiten eines Kühl-oder Heizfluids durch das Modul und insbesondere die Walze vorgesehen. In alternativer Ausgestaltung wird die Walzenoberfläche lediglich über Wärmeleitung über das von außen insgesamt temperierte Modul temperiert. In alternativer Ausgestaltung wird das zugeführte zu bestrahlende Medium temperiert, vorzugsweise vor dem Eintritt in das Modul, alternativ oder zusätzlich während der Bestrahlung.

Erfindungsgemäß ist mindestens ein Strahlungsfenster an dem Gehäusedeckel der Kassette vorgesehen, um das Modulgehäuse gas- und luftdicht zu verschließen. In einer bevorzugten Ausgestaltung ist zum Schutz der Strahlungsquelle ein Strahlungsfenster auch an der Anordnung, woran die Kassette ankoppelbar ist, vorgesehen. Art und Ausgestaltung des Strahlungsfensters ist von der Art und Qualität der Bestrahlung abhängig. Für eine Bestrahlung mit ionisierender kurzwelliger Strahlung, UV-C oder weicher Röntgenstrahlung, können Strahlungsfenster aus Kunststoff oder Quarzglas eingesetzt werden. Erfindungsgemäß aber sind - zur Bestrahlung mit Röntgenstrahlung oder Betastrahlung - Metallfenster vorgesehen. Metalle der Strahlungsfenster sind bevorzugt ausgewählt aus Titan, Magnesium und Aluminium sowie Legierungen davon.

Vorzugsweise wird das Modulgehäuse vor oder während des Betriebs gekühlt, beispielsweise temperiert auf 4°C. Dabei kann bei gegebener Umgebungsfeuchte an dem Strahlungsfenster Flüssigkeit kondensieren. In einer bevorzugten Ausgestaltung ist daher das Strahlungsfenster mit trockenem Gas überspülbar um eine Taubildung dort zu verhindern. Vorzugsweise dient das trockene Gas gleichzeitig zur Kühlung des Strahlungsfensters während der Bestrahlung.

In einer besonderen Variante wird die Rotation der Walze in dem Modul über den Druck und Fluss der den Modul zum Zwecke der Bestrahlung zugeführten Flüssigkeit vermittelt. Die Flüssigkeit, welche über den mindestens einen Zulaufkanal zugeführt wird, treibt dabei die Rotation der Walze in einer ersten Variante im Hauptstrom an; das heißt, sämtliche zugeführte Flüssigkeit, die zu einem kontinuierlichen Flüssigkeitsfilm "gewandelt" wird, dient dem Antrieb der Walze. Eine bevorzugte Ausgestaltung dieser Variante ist ein an der Walzenachse oder in der Walze angeordnetes Turbinen- oder Flügelradelement, durch welches die gesamte zugeführte Flüssigkeit strömt. In einer weiteren solchen Variante ist die Walzenoberfläche gegenüber der zugeführten Flüssigkeit derart "haftend" ausgebildet, dass die über das spaltbildende Element auf die Walzenoberfläche auflaufende Flüssigkeit aufgrund der dort herrschenden Druck- und Schwerkraftverhältnisse die Walze reibungsgetrieben in Bewegung setzt.

In einer alternativen Variante treibt die zugeführte Flüssigkeit die Walze im Nebenstrom an, wobei ein Teil der zugeführten Flüssigkeit in den kontinuierlichen Flüssigkeitsfilm "gewandelt" wird, ein anderer Teil aber zum Antrieb der Walze dient und in die Wanne zurückfließt. In dieser Variante ist bevorzugt an ein oder mehreren Positionen entlang der Mantelfläche der Walze eine umlaufende Schaufelradstruktur vorgesehen, worauf ein Teilstrom der zugeführten Flüssigkeit gerichtet ist, um die Walze in Rotation zu versetzen. Dabei ist besonders vorgesehen, dass dieser Teil der zugeführten Flüssigkeit keinen kontinuierlichen Flüssigkeitsfilm bildet und auch nicht von der vorgesehenen Abstreifkante an der ablaufenden Seite der Walze abgestreift und über den Ablaufkanal des Moduls abgeleitet wird. Die Abstreifkante weißt dazu an der Stelle der umlaufenden Schaufel eine Ausnehmung auf, die dort ein Abstreifen von Flüssigkeit verhindert.

In alternativen oder zusätzlichen Ausführungen ist die Walze des Moduls durch einen externen Antrieb in Rotation versetzbar. In einer einfachen Ausgestaltung ist dazu die Achswelle der Walze des Moduls aus dem Modulgehäuse nach außen geführt und kann dort mit einem externen Antrieb, beispielsweise einem Getriebemotor oder Schrittmotor, über eine geeignete mechanische Kupplung in Eingriff kommen. In einer Ausführung ist die Walzenachse an dem Modulgehäuse direkt nach Außen geführt. In einer alternativen Ausgestaltung ist die Antriebswelle, die aus dem Modulgehäuse führt, innerhalb des Modulgehäuses über ein Getriebe, bevorzugt Spindelgetriebe, Zahnräder oder Zahnriemen, mit der Walzenachswelle gekoppelt.

Die Wellendurchführung der Antriebswelle oder Walzenachswelle ist bevorzugt als doppelte Wellendichtung mit vorzugsweise spülbarem Zwischenraum in dem Modulgehäuse ausgebildet. Der Zwischenraum kann mit aseptischem und/oder desinfizierendem Spülmedium gespült werden.

In einer alternativen und bevorzugten Ausgestaltung erfolgt die Drehmomentkopplung der Walze zu dem externen Antriebselement berührungslos magnetisch, insbesondere über in die Walze eingesetzte Magnetelemente, die zusammen mit einer entsprechenden Struktur an dem Antriebselement eine Magnetkupplung bilden. In einer bevorzugten Ausgestaltung dieser Variante ist vorgesehen, dass die Magnetelemente der Walze mit einem externen elektromagnetischen Antrieb, besonders einer feststehenden, wechselnd strombeaufschlagten Magnetspulenanordnung, in Kontakt gebracht werden können, um zusammen einen Elektromotor zu bilden.

Die erfindungsgemäße auswechselbare Kassette kann in eine feststehende Apparatur, beispielsweise innerhalb von Automatenstraßen, eingesetzt werden und kann dabei nach Bedarf, zum Zwecke der Reinigung oder Sterilisierung, ausgetauscht werden. Es ist vorgesehen, dass die Kassette dabei sämtliche Anschlüsse zur Zu- und Ableitung der biologischen Flüssigkeit aufweist. Diese können mit Schnellwechseladaptern versehen sein. In einer bevorzugten Ausführung sind die Schnellwechselverbindungen für die Flüssigkeitszuläufe und -abläufe derart am den Modulgehäuse angeordnet, dass mit dem Einlegen oder Einschieben der Kassette in die Sterilisationsanordnung die Fluidverbindungen selbsttätig hergestellt werden. Solche Verbindungen können als CIP "clean-in-place", WIP "wash-in-place" oder SIP "sterilization-in-place" ausgelegt sein. Alternative an sich bekannte Verbindungen sind Ventile und Sterilverbinder wie Luer-Lock und verwandte Systeme.

Die erfindungsgemäße auswechselbare Kassette selbst besitzt vorzugsweise einen modularen Aufbau, sodass Elemente, welche innerhalb des Moduls die Ausbildung, besonders die Dicke, des gewünschten Flüssigkeitsfilms bestimmen, einzeln ausgetauscht werden können. Beispielsweise können mehrere verschiedene dimensionierte spaltbildende Elemente bereitgestellt werden, die in der Art eines Baukastensystems an dem Modul ausgewechselt werden können, um die Dicke des Flüssigkeitsfilms anzupassen. Ebenso kann die Walze selbst in verschiedenen Varianten bereitgestellt werden, wobei sich die Varianten im Wesentlichen durch Art und Beschaffenheit der Walzenoberfläche unterscheiden. Durch den modularen Aufbau, insbesondere die Ausgestaltung des Moduls als auswechselbare und geschlossene Kassette, ist es möglich, ein Gesamtsystem für die Inaktivierung an sich gefährlicher Erreger auch in Laboren und Automatenstraßen geringerer Sicherheitsstufen zu betreiben. Durch die mögliche voll geschlossene Ausführung wird es möglich, das Modul als Ganzes auch inklusive der notwendigen Zu- und Ableitungen der Flüssigkeit und der dazugehörigen Vorlagengefäße in einem ersten Labor höherer Sicherheitsstufe zu beschicken und vorzubereiten und diese Anordnung dann steril geschlossen in die Automatenstraße geringerer Sicherheitsstufe zu verbringen, wo dann die Strahlungsbehandlung zur Inaktivierung der pathogenen Erreger erfolgen kann. Durch die vollgeschlossene Ausführung des Moduls kann die Kontaminierung der übrigen Automatenteile, insbesondere der Strahlungsquelle, der Antriebe der Walze und der Pumpen verhindert werden. Gleichzeitig wird Personenschutz und Produktschutz erreicht oder verbessert.

In einer ersten Variante ist das Modul einseitig offen und erlaubt freie Projektion, der thermischen oder ionisierenden Strahlung auf den auf der Walzenoberfläche exponierten Flüssigkeitsfilm. Dabei ist bevorzugt vorgesehen, dass dieses Modul zum Betrieb in eine feststehende Anordnung gesteckt oder geschoben oder anderweitig verkoppelt wird und mit dieser dicht verschlossen wird. Diese feststehende Anordnung enthält ihrerseits zumindest die Strahlungsquelle und vorzugsweise zusätzlich Mittel zum Transport der Flüssigkeit durch das Modul und/oder zusätzlich Mittel zum Antrieb der Rotation der Walze in dem Modul.

In einer alternativen Ausgestaltung ist das Modul in sich voll geschlossen und wird bevorzugt als austauschbare standardisierte Kassette bereitgestellt. Zum Verschluss des Modulgehäuses dient bevorzugt ein dichter Gehäusedeckel, wobei der Gehäusedeckel insbesondere ein gas- und flüssigkeitsdichtes aber strahlungsdurchlässiges Strahlungsfenster aufweist, zum Zwecke der Bestrahlung des auf der Walzenoberfläche gebildeten Flüssigkeitsfilms mittels einer externen Strahlungsquelle durch das Fenster.

Gegenstand der Erfindung ist auch eine Vorrichtung zur kontinuierlichen dosiskontrollierten Bestrahlung von Flüssigkeit, insbesondere eines biologischen Mediums, beispielsweise zur Inaktivierung von Pathogenen in dem Medium oder zur Modifikation von Komponenten des Mediums mittels ionisierender oder thermischer Strahlung, welche das erfindungsgemäße Modul und zusätzlich mindestens eine Strahlungsquelle enthält, wobei insbesondere das Modul an die Strahlungsquelle unmittelbar koppelbar ist. Weiter weist die Vorrichtung zumindest eine oder mehrere Pumpen zum kontinuierliche aktiven Transport dieser Flüssigkeit durch das Modul auf.

In einer Variante ist zusätzlich zumindest ein Vorlagengefäß, enthaltend die zu bestrahlende Flüssigkeit und mindestens ein Auffanggefäß zur Aufnahme der bestrahlten Flüssigkeit, welche aus dem Modul strömt, vorhanden. In einer bevorzugten Ausgestaltung wird das Vorlagengefäß über eine erste Zulaufleitung mit dem Zulauf zur kontinuierlichen Beschickung des Moduls mit dieser Flüssigkeit vorgesehen. Der Überlauf des Moduls mündet vorzugsweise in das Vorlagengefäß. Der Transport der Flüssigkeit in das Modul erfolgt aktiv, insbesondere durch Druckbeaufschlagung des Vorlagengefäßes, vor allem wenn der Überlauf nicht wieder im Vorlagengefäß endet, und/oder durch Pumpenelemente in dem Zulauf. Der Transport der Flüssigkeit aus dem Überlauf des Moduls in das Vorlagengefäß erfolgt passiv, insbesondere gestützt durch den entstehenden Unterdruck im Vorlagengefäß, der bei dem Absaugen der zuzuführenden Flüssigkeit aus dem Vorlagengefäß entsteht, und/oder schwerkraftgestützt. Alternativ und bevorzugt erfolgt der Transport der Flüssigkeit aktiv über eine Pumpe, insbesondere Schlauchpumpe.

Das Aufnahmegefäß zur Aufnahme der bestrahlten Flüssigkeit ist mit dem Abflaufkanal des Moduls verbunden. In bevorzugter Ausgestaltung ist zusätzlich ein Druck ausgleichender Entlüftungskanal vorgesehen, welcher die Ablaufsseite des Moduls mit dem Ablaufgefäß verbindet. Der Abtransport der abgestreiften bestrahlten Flüssigkeit erfolgt vorzugsweise passiv, insbesondere schwerkraftgestützt, alternativ durch aktiven Transport, durch Druck- oder Vakuumbeaufschlagung des Ablaufgefäßes und/oder durch aktive Pumpelemente im Ablaufzweig des Moduls.

Bevorzugt enthält diese Vorrichtung zusätzlich zumindest ein mechanisches oder elektromagnetisches Antriebselement, gegebenenfalls mit geeigneten mechanischen oder kontaktlosen Koppelelementen zum Antrieb der Rotationen der Walze in dem Modul.

Das erfindungsgemäße Modul und die erfindungsgemäße Vorrichtung sind spezifisch geeignet zur Vergleichmäßigung kontinuierlich zugeführter Flüssigkeit zum Zwecke der dosiskontrollierten Bestrahlung der Flüssigkeit. Dies dient wie hierin beschrieben vornehmlich zur gezielten Inaktivierung von pathogenen in der Flüssigkeit. Demgemäß ist ein weiterer Gegenstand der Erfindung ein Verfahren zur Inaktivierung von Pathogenen in einer biologischen Flüssigkeit, welches insbesondere kontinuierlich durchgeführt wird. Das erfindungsgemäße Verfahren enthält zumindest die folgenden Schritte: In Schritt (a) wird die Flüssigkeit, welche gegebenenfalls aktive Pathogene enthält, zu dem erfindungsgemäßen Modul insbesondere aktiv zugeführt. In Schritt (b) wird die Walze in dem erfindungsgemäßen Modul rotiert, sodass sich ein kontinuierlicher Flüssigkeitsfilm vorbestimmbarer Dicke aus der zugeführten biologischen Flüssigkeit auf der umlaufenden Walzenoberfläche bildet. In Schritt (c) wird der auf der Walzenoberfläche gebildete und exponierte Flüssigkeitsfilm mit einer pathogeninaktivierenden Dosis ionisierender Strahlung bestrahlt, wobei die Strahlungsdosis durch die Strahlungsintensität der Strahlungsquelle, die durch den strahlungsexponierten Volumenabschnitt der Flüssigkeit, welcher durch das Strahlungsfenster und die Dicke des gebildeten Flüssigkeitsfilms bestimmt wird, sowie vorzugsweise zusätzlich durch die Durchfluss- oder Strömungsgeschwindigkeit des Flüssigkeitsfilms, welcher aber die Rotationsgeschwindigkeit der Walze bestimmbar ist. In Schritt (d) wird die nach Durchlaufen des Strahlungsfensters bestrahlte und von der Walzenoberfläche abgestreifte Flüssigkeit aus dem Modul abgeführt und aufgenommen. Diese Flüssigkeit enthält dosisabhängig inaktivierte Pathogene.

Ein erfindungsgemäßes Verfahren betrifft auch allgemein die thermische oder ionisierende Bestrahlung einer Flüssigkeit mittels des erfindungsgemäßen Moduls, enthaltend die Schritte (a): Zuführen der Flüssigkeit zu dem Modul, (b) Rotieren der Walze in dem Modul, sodass ein kontinuierlicher Flüssigkeitsfilm bestimmbarer Dicke auf der umlaufenden Walzenoberfläche gebildet wird, (c) Bestrahlen des Flüssigkeitsfilms auf der Walzenoberfläche mit thermischer oder ionisierender Strahlung, und (d) Aufnahmen der aus dem Modul aufführbaren bestrahlten Flüssigkeit.

Bevorzugt wird die Strahlungsdosis durch die Strahlungsquelle und die Rotationsgeschwindigkeit eingestellt. Da innerhalb der Flüssigkeit ein Dosisgefälle entsteht, ist es in vielen Fällen besonders erstrebenswert, eine möglichst geringe Schichthöhe des Flüssigkeitsfilms an der Walze auszubilden; dies bedingt vorteilhafterweise geringe Unterschiede in der Strahlungsdosis innerhalb der transportierten Flüssigkeit. Die Einstellung der Schichthöhe (größer als minimal möglich), ist vor dem Hintergrund eines höheren Durchsatzes erstrebenswert, für den Fall, dass der Dosisgradient in der Flüssigkeit vertretbar ist. Dabei ist bevorzugt vorgesehen, dass die Dicke des Flüssigkeitsfilms über die Einstellung des Kapillarspalts an dem Ablaufelement des Moduls, gegebenenfalls in Interaktion mit einem zusätzlich vorgesehenen Abstreifelement, gesteuert wird. Alternativ oder vorzugsweise zusätzlich wird die Dosis durch die Rotationsgeschwindigkeit der Walze bestimmt und gesteuert - die Rotationsgeschwindigkeit bestimmt maßgeblich die Verweildauer eines bestimmten Volumenabschnitts des Mediums in dem bestrahlten Bereich. Die Rotationsgeschwindigkeit bestimmt gegebenenfalls auch die Dicke des Flüssigkeitsfilms. Daneben ist vorgesehen, die Dosis und Eindringtiefe durch direkte Steuerung der Strahlungsquelle, z.B. der Beta-Strahlungsquelle, in an sich bekannter Weise zu bestimmen.

Ein weiterer Gegenstand der Erfindung ist die Verwendung des Moduls oder der Vorrichtung dieser Erfindung zur kontinuierlichen Inaktivierung von Pathogenen in einem flüssigen biologischen Medium, vorzugsweise einer Virussuspension, mittels ionisierender Strahlung.

Schließlich ist ein weiterer Gegenstand der Erfindung die Verwendung des Moduls oder der Vorrichtung dieser Erfindung zur Modifikation biologischer Medien mittels ionisierender Strahlung.

Die Erfindung wird durch die nachfolgenden Figuren und Ausführungsbeispiele näher erläutert:

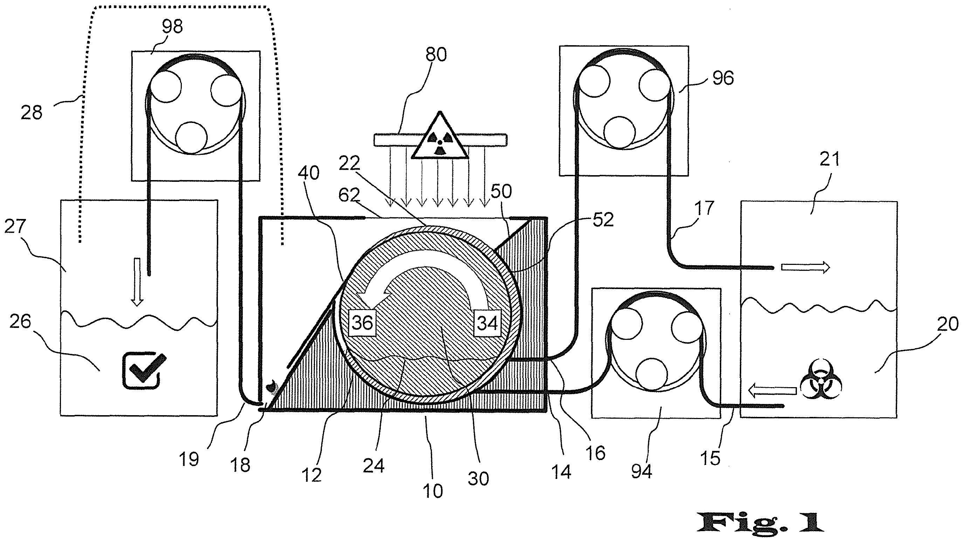

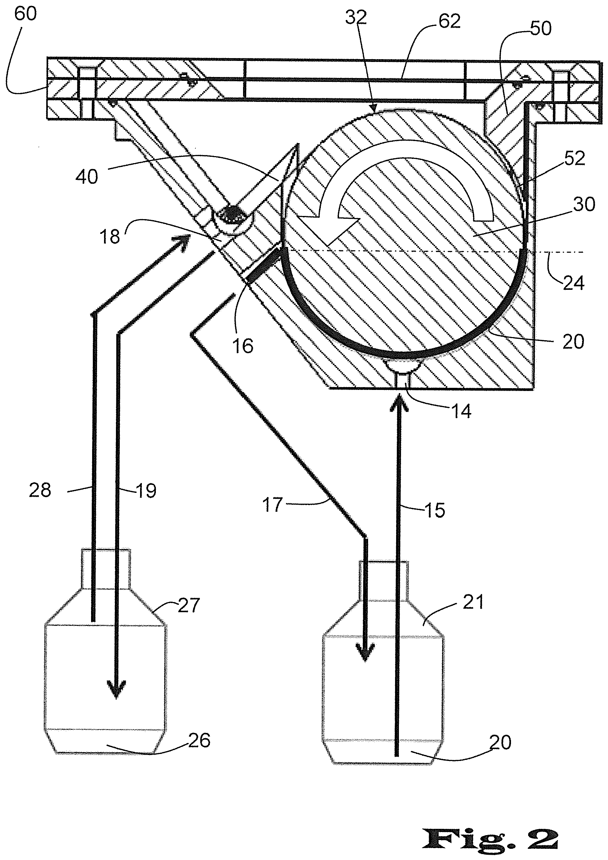

Figur 1 zeigt schematisch den Gesamtaufbau zur kontinuierlichen Bestrahlung von Pathogen enthaltenden Flüssigkeiten zur Inaktivierung der Pathogene unter Verwendung des erfindungsgemäßen Moduls: In einem Vorlagengefäß 21 ist ein gegebenenfalls Pathogene enthaltendes flüssiges Medium 20 vorgelegt. Dies wird aktiv über die Zuleitung 15 und die Peristaltikpumpe 94 über den Zulauf 14 an dem Modulgehäuse 10 in die Wanne 12 geführt. Ein in dem Modulgehäuse vorgesehener Überlauf 16 führt über Leitung 17 und eine optionale Peristaltikpumpe 96 überschüssige Flüssigkeit in das Vorlagengefäß 21 zurück. Der Flüssigkeitspegel 24 in der Wanne 12 wird konstant gehalten. In der Flüssigkeit 20 in der Wanne 12 rotiert die Zylinderwalze 30. Das erfindungsgemäß auf der bei Rotation auflaufenden Seite 34 der Walze angeordnete spaltbildende Element 50 bildet einen Kapillarspalt 52 zu der Walzenoberfläche, wobei der Kapillarspalt 52 sich auch oberhalb des Flüssigkeitspegels 24 erstreckt. Bei Rotation der Walze 30 vermittelt der Kapillarspalt 52 die Ausbildung und Vergleichmäßigung eines Flüssigkeitsfilms 22 auf der Walzenoberfläche. Der gebildete Flüssigkeitsfilm 22 wird an einem Strahlungsfenster 62 vorbeigeführt und ist dort der Strahlung einer Strahlungsquelle 80 exponiert. Der Flüssigkeitsfilm 22 wird nach der Bestrahlung auf der ablaufenden Seite 36 der Walze 30 von einer Abstreifkante 40, welche dort dichtend an der Walzenoberfläche anliegt, im Wesentlichen vollständig abgezogen oder abgestreift. Die abgezogene bestrahlte Flüssigkeit 26 wird gesammelt und über den Ablaufkanal 18 über Leitung 19, gegebenenfalls über Peristaltikpumpe 98 in ein Auffanggefäß 27 abgezogen. Eine optionale Druckleitung 28 stellt Druckausgleich her. Figur 2 zeigt die Prinzipverschaltung des Flüssigkeitsstroms in der Gesamtanordnung analog zu Figur 1 anhand der schematischen Schnittzeichnung einer spezifischen Ausführung des erfindungsgemäßen Moduls.

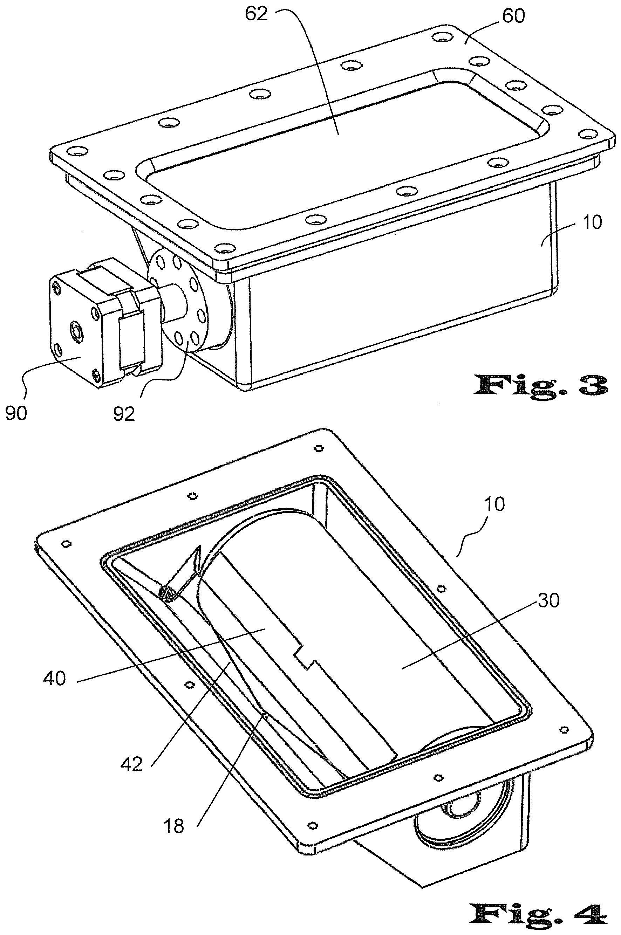

Figur 3 zeigt eine perspektivische Draufsicht einer spezifischen Ausführung des erfindungsgemäßen Moduls 10 mit dicht aufgesetztem Deckel 60 mit Strahlungsfenster 62. An dem Modul ist ein Antriebselement 90 mit Kupplungselement 92 dargestellt zum Antrieb der in dem Modul enthaltenen Walze. Der Antrieb der Walze in dem Modul 10 erfolgt hier berührungslos über Magnetelemente in dem Kupplungselement 92 und entsprechenden Magnetelementen in der Walze. Figur 4 zeigt eine perspektivische Ansicht des Moduls 10 gemäß Figur 3 mit entferntem Deckel und Blick auf die rotierbare Zylinderwalze 30, der an der Oberfläche der Zylinderwalze 30 anliegenden Abstreifkante 40, hier in Form eines abfedernd anliegenden Abstreifblechs, mit einer zu dem Ablaufkanal 18 mündenden Auffangrinne 42.

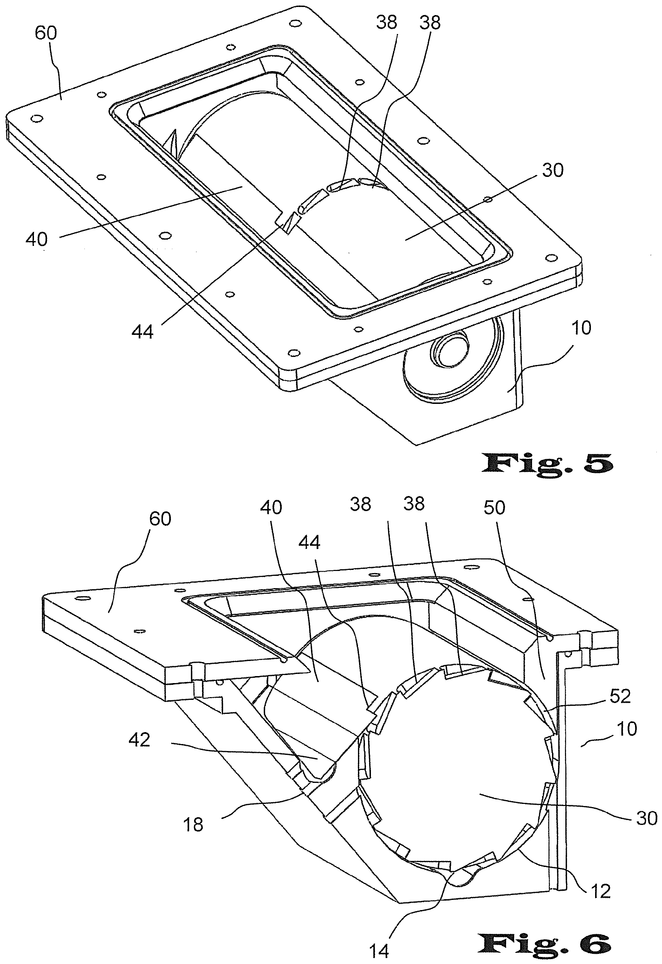

Figur 5 zeigt eine alternative Ausgestaltung eines erfindungsgemäßen Moduls 10 mit Deckel 60, hier mit optisch durchsichtigem Strahlungsfenster. Die in der Wanne des Modulgehäuses rotierbare Zylinderwalze 30 weist zumindest einen umlaufenden Schaufelradring mit Schaufelelementen 38 auf, welche von dem Modul zugeführter Flüssigkeit gespeist werden, um die Walze 30 in Rotation zu versetzen. In dieser spezifischen Ausgestaltung weist die Abstreifkante 40 an der Stelle der umlaufenden Schaufelelemente 38 eine Ausdehnung 44 auf, um dort umlaufende Flüssigkeit, welche keinen definierten Flüssigkeitsfilm bildet, nicht abzustreifen. Figur 6 zeigt eine Schnittansicht der spezifischen Ausführung gemäß Figur 5. An der Wanne 12, worin die Zylinderwalze 30 läuft, ist ein Zulaufkanal 14 ausgebildet sowie ein Überlaufkanal 16. In der dargestellten spezifischen Ausgestaltung ist das spaltbildende Element 50 zur Ausbildung des Kapillarspalts 52 bevorzugt einstückig mit dem auf das Modulgehäuse aufsetzbaren Deckel 60 ausgebildet. Auf der ablaufenden Seite der Walze 30 ist die Abstreifkante 40 mit Ausnehmung 44 in Form eines federnd auf der Walzenoberfläche aufliegenden Abstreifblechs ausgebildet. Die von der Walzenoberfläche abgestreifte Flüssigkeit wird in der Auffangrinne 42 gesammelt und über den Ablaufkanal 18 aus dem Modul abgeführt.

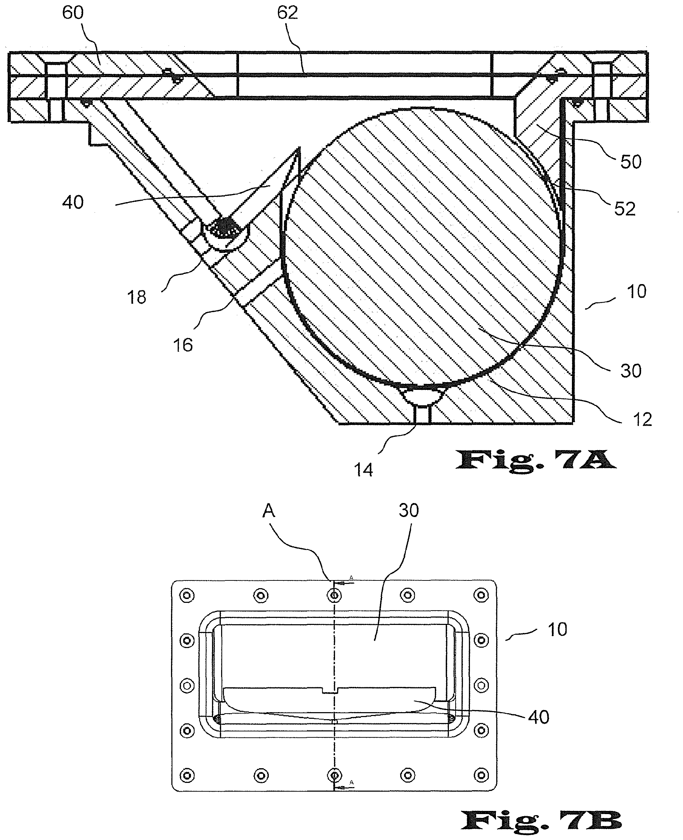

Figuren 7A und 7B sind schematische Schnittansichten einer spezifischen Ausgestaltung des Moduls 10 gemäß Figur 2. Figur 7 B zeigt eine Draufsicht auf dasselbe Modul als Ganzes mit eingezeichneter Schnittlinie A, welche die Schnittebene in den korrespondierenden Figuren 7A, 6 und 2 bezeichnet.

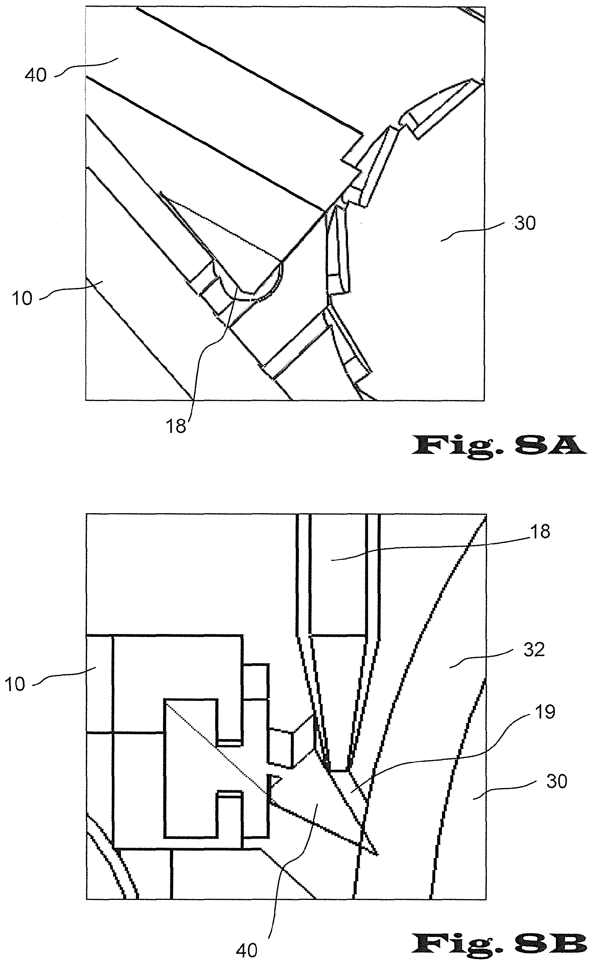

Figuren 8A und 8B sind schematische Schnittansichten von Ausschnitten aus den in den Figuren 4 und 5 beziehungsweise 9 und 10A und 10B dargestellten Ausführungen. Figur 8A zeigt eine erste Ausführung und Anordnung der Abstreifkante 40 an der Walze 30. Der Abstreifer 40 ist entgegen der Rotationsrichtung der Walze 30 gerichtet. Figur 8B zeigt eine dazu alternative Ausführung und Anordnung der Abstreifkante 40. Der als Abstreiflippe ausgebildete Abstreifer 40 ist in Rotationsrichtung der Walze 30 gerichtet. Im Falle der Ausführung gemäß Figur 8A kann die mit dem Abstreifer 40 von der Walze 30 abgestreifte Flüssigkeit über den Abstreifer hinweg in den als Rinne ausgebildeten Ablauf 18 fließen. Im Falle der Ausführung gemäß Figur 8B kann die mit der Abstreiflippe 40 von der Walze 30 abgestreifte Flüssigkeit in eine zwischen Walzenoberfläche und Abstreiflippe 40 gebildete Rinne 19 fließen und kann von dort über den als Kanüle ausgebildeten Ablauf 18 aktiv oder passiv abgezogen werden.

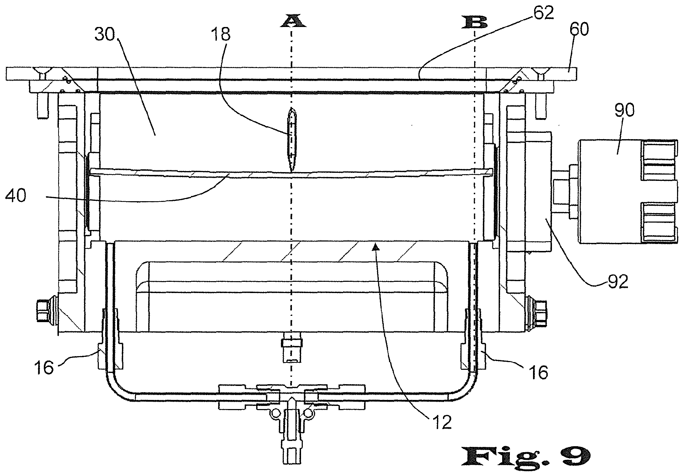

Figur 9 zeigt in schematischer Querschnittsansicht eine weitere Ausführung der Kassette. Die Schnittebene der Ansicht befindet sich im Bereich der Abstreiflippe 40. Das Modulgehäuse 10 ist mit dem Gehäusedeckel 60 verschlossen. In dem Gehäusedeckel 60 befindet sich das Strahlungsfenster 62. Die Abstreiflippe 40 vor der Walze 30 verläuft in erfindungsgemäß bevorzugter Ausführung bogenförmig, sodass, schwerkraftgestützt, von der Walze 30 abgestreifte Flüssigkeit sich primär im Bereich des Ablaufrohrs 18, das bevorzugt mittig angeordnet ist, sammelt. In der dargestellten Ausführung wird die Rotation der Walze über eine axial angeordnete Kupplung 92 mit der durch das Gehäuse 10 durchgeführten Welle und der Antriebseinheit 90 vermittelt. In der dargestellten Ausführung sind die Überlaufrohre 16, welche in die Wanne 12 ragen, bevorzugt optional in der Eintauchtiefe verstellbar, wodurch der Flüssigkeitsstand in der Wanne 12 voreingestellt werden kann.

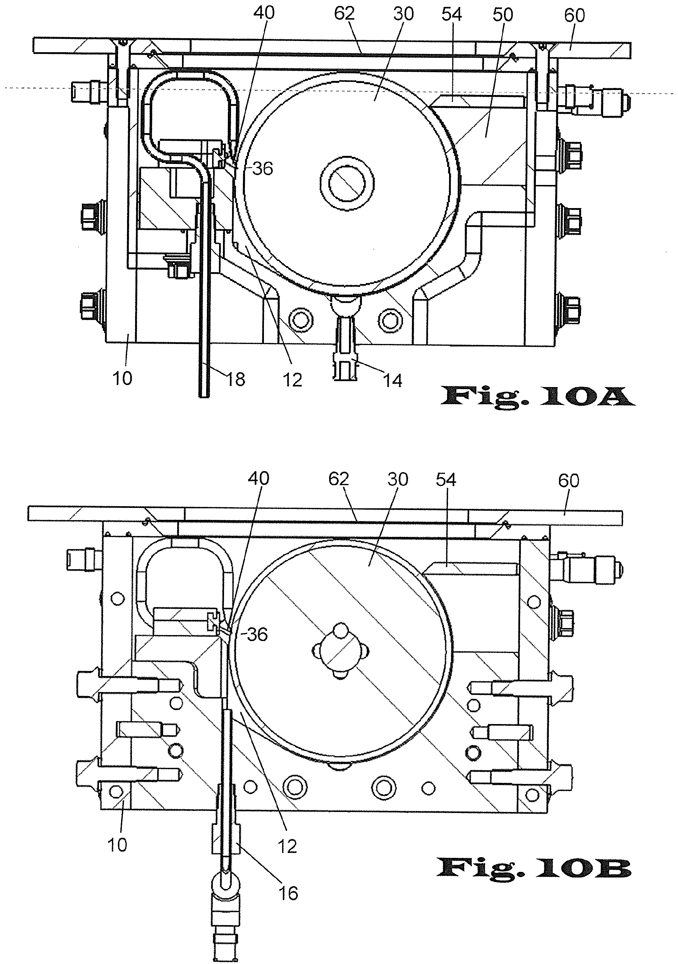

Figuren 10A und 10B zeigen schematische Schnittdarstellungen der Ausführung nach Figur 9. Die Schnittebene der Figur 10A ist als Linie "A" in Figur 9 dargestellt. Die Schnittebene der Figur 10B ist als Linie "B" in Figur 9 dargestellt. Die Bezugszeichen gelten entsprechend. Der Zulauf 14 der Flüssigkeit befindet sich auf der Unterseite der Wanne 12. Der Ablauf 18 ragt in die Zwischenwalze 30 und Abstreiflippe 40 gebildete Rinne. Das Überlaufrohr 16 kann in der Höhe variiert werden, um den Flüssigkeitspegel in der Wanne 12 zu bestimmen.

## Patentansprüche

1. Kassette zur Erzeugung eines kontinuierlichen Flüssigkeitsfilms (22) aus zugeführter biologischer Flüssigkeit (20), geeignet für die Bestrahlung des erzeugten Flüssigkeitsfilms (22) und kontinuierliche Inaktivierung von Pathogenen in der biologischen Flüssigkeit in einer Anordnung zur kontinuierlichen dosiskontrollierten Bestrahlung,
mit einem Modulgehäuse (10) enthaltend:
Wanne (12) zur Aufnahme der Flüssigkeit (20),
Zulaufkanal (14) zur Zufuhr der Flüssigkeit (20) in die Wanne (12),
Zylinderwalze (30), die in die Wanne (12) und Flüssigkeit (20) eintaucht und dort rotierbar ist,
Abstreiflippe (40) auf der ablaufenden Seite (36) der Walze (30) in dichtem Kontakt mit der Walzenoberfläche (32) zum Abstreifen des bei Rotation der Walze (30) auf der umlaufenden Walzenoberfläche (32) gebildeten Flüssigkeitsfilms (22) und
Ablaufkanal (18) zur Aufnahme und Ableitung von mit der Abstreiflippe (40) abgestreifter Flüssigkeit (26), und
mit einem Gehäusedeckel (60) zum dichten Verschließen des Modulgehäuses (10),
**dadurch gekennzeichnet, dass** der Gehäusedeckel (60) ein gas- und flüssigkeitsdichtes, und für Betastrahlung durchlässiges Metallfenster (62) aufweist,
dass das Modulgehäuse (10) einen Überlaufkanal (16) zum Ablauf von überschüssiger Flüssigkeit aus der Wanne (12), um einen Flüssigkeitspegel (24) in der Wanne (12) festzulegen, enthält, und
dass die Kassette, sterilisierbar und zur wiederkehrenden Verwendung an der Anordnung zur kontinuierlichen dosiskontrollierten Bestrahlung austauschbar ist.

2. Kassette nach Anspruch 1, wobei das Modulgehäuse (10) weiter enthält:
spaltbildendes Element (50) auf der auflaufenden Seite (34) der Walze (30) zur Ausbildung und Vergleichmäßigung des Flüssigkeitsfilms (22) auf der Walzenoberfläche (32), wobei das spaltbildende Element (50) auf der auflaufenden Seite (34) der Walze (30) zu der Walzenoberfläche (32) so beabstandet ist, um einen Kapillarspalt (52) zu bilden, wobei sich der Kapillarspalt (52) bis oberhalb des Flüssigkeitspegels (24) erstreckt.

3. Kassette nach Anspruch 2, wobei das spaltbildende Element (50) in seinem Abstand zu der Walzenoberfläche (32) einstellbar oder auswechselbar ist, um die Dicke des dort gebildeten Flüssigkeitsfilms (22) zu steuern.

4. Kassette nach einem der vorstehenden Ansprüche, wobei die Abstreiflippe (40) an der ablaufenden Seite (36) der Walze (30) entgegen die Ablaufrichtung der rotierenden Walze gerichtet ist.

5. Kassette nach einem der Ansprüche 1 bis 3, wobei die Abstreiflippe (40) an der ablaufenden Seite (36) der Walze (30) in Ablaufrichtung der rotierenden Walze gerichtet ist.

6. Kassette nach Anspruch 5, wobei der Ablaufkanal (18) als mindestens ein in die zwischen Abstreiflippe (40) und der ablaufenden Seite (36) der Walze (30) gebildete Rinne (58) ragendes Rohr ausgebildet ist.

7. Kassette nach einem der vorstehenden Ansprüche, wobei das Modulgehäuse (10) eine Kupplung (92) zum externen Antrieb der Rotation der Walze (30) über ein außerhalb des Modulgehäuses (10) angeordnete koppelbare Antriebseinheit (90) ausweist.

8. Kassette nach Anspruch 7, wobei die Walze (30) Magnetelemente aufweist zum externen Rotationsantrieb der Walze (30) über ein außerhalb des Modulgehäuses (10) angeordnetes und mit der Walze (30) über die Kupplung (92) magnetisch koppelbares mechanisches oder elektromagnetisches Antriebseinheit (90).

9. Anordnung zur kontinuierlichen dosiskontrollierten Bestrahlung von biologischer Flüssigkeit (20) für die kontinuierliche Inaktivierung von Pathogenen in der biologischen Flüssigkeit (20), enthaltend:
die austauschbare Kassette nach einem der vorstehenden Ansprüche und
eine Quelle (80) für Betastrahlung,
wobei die Kassette unmittelbar an die Strahlungsquelle (80) gekoppelt ist.

10. Anordnung nach Anspruch 9, weiter enthaltend eine oder mehrere Pumpen (94,96) zum kontinuierlichen aktiven Transport der Flüssigkeit (20,26) durch das Modulgehäuse (10).

11. Anordnung nach Anspruch 9 oder 10, weiter enthaltend eine mechanisches oder elektromagnetische Antriebseinheit (90) zum Rotationsantrieb der Walze (30) in dem Modul (10).

12. Verfahren zur Inaktivierung von Pathogenen in einer biologischen Flüssigkeit (20), enthaltend die Schritte:
a) Zuführen der biologischen Flüssigkeit (20) gegebenenfalls enthaltend aktive Pathogene zu der Kassette nach einem der Ansprüche 1 bis 8,
b) Rotieren der Walze (30) in dem Modulgehäuse (10) der Kassette, so dass ein kontinuierlicher Flüssigkeitsfilm (22) der biologischen Flüssigkeit (20) mit vorbestimmbarer Dicke auf der umlaufenden Walzenoberfläche (32) gebildet wird,
c) Bestrahlen des Flüssigkeitsfilms (22) auf der Walzenoberfläche (32) mit ionisierender Betastrahlung in einer Dosis, welche die Inaktivierung der Pathogene der biologischen Flüssigkeit (20) bewirkt,
d) Aufnehmen der bestrahlten Flüssigkeit (26) mit inaktiven Pathogenen von der Walzenoberfläche (32).

13. Verwendung der Kassette nach einem der Ansprüche 1 bis 8 zur kontinuierlichen Inaktivierung von Pathogenen in einer biologischen Flüssigkeit mittels ionisierender Betastrahlung.

## Claims

1. A cassette for generating a continuous liquid film (22) from supplied biological liquid (20), suitable for irradiation of the generated liquid film (22) and continuous inactivation of pathogens in the biological liquid in a continuous dose-controlled irradiation assembly
with a module housing (10) containing:
Tub (12) for receiving the liquid (20),
feed channel (14) for feeding the liquid (20) into the tub (12),
cylindrical roller (30) which is immersed in the tub (12) and liquid (20) and is rotatable there,
wiper lip (40) on the outgoing side (36) of the roller (30) in tight contact with the roller surface (32) for wiping off the liquid film (22) formed on the rotating roller surface (32) during rotation of the roller (30), and
a discharge channel (18) for receiving and discharging liquid (26) wiped off by the wiper lip (40), and
with a housing cover (60) for tightly closing the module housing (10),
**characterized in that** the housing cover (60) has a gas- and liquid-tight metal window (62) which is permeable to beta radiation,
**in that** the module housing (10) includes an overflow channel (16) for draining excess liquid from the tub (12) to establish a liquid level (24) in the tub (12), and
**in that** the cassette, is sterilisable and replaceable for recurrent use on the continuous dose controlled irradiation assembly.

2. The cassette of claim 1, wherein the module housing (10) further contains:
a gap-forming element (50) on the upstream side (34) of the roller (30) for forming and uniforming the liquid film (22) on the roller surface (32), wherein the gap-forming element (50) on the upstream side (34) of the roller (30) is spaced from the roller surface (32) so as to form a capillary gap (52), the capillary gap (52) extending to above the liquid level (24).

3. The cassette according to claim 2, wherein the gap-forming element (50) is adjustable or replaceable in its spacing from the roller surface (32) to control the thickness of the liquid film (22) formed thereat.

4. The cassette according to any one of the preceding claims, wherein the wiping lip (40) on the outgoing side (36) of the roller (30) is directed opposite to the outgoing direction of the rotating roller.

5. The cassette according to any one of claims 1 to 3, wherein the wiper lip (40) on the outgoing side (36) of the roller (30) is directed in the outgoing direction of the rotating roller.

6. The cassette according to claim 5, wherein the discharge channel (18) is formed as at least one tube projecting into the groove (58) formed between the wiper lip (40) and the outgoing side (36) of the roller (30).

7. The cassette according to any one of the preceding claims, wherein the module housing (10) has a coupling (92) for externally driving the rotation of the roller (30) via a couplable drive unit (90) arranged outside the module housing (10).

8. The cassette according to claim 7, wherein the roller (30) has magnetic elements for externally driving the rotation of the roller (30) via a mechanical or electromagnetic drive unit (90) arranged outside the module housing (10) and magnetically couplable to the roller (30) via the coupling (92).

9. Assembly for the continuous dose-controlled irradiation of biological fluid (20) for the continuous inactivation of pathogens in the biological fluid (20), containing:
the replaceable cassette of any one of the preceding claims; and
a source (80) of beta radiation,
wherein the cassette is directly coupled to the source of radiation (80).

10. The assembly according to claim 9, further having one or more pumps (94,96) for continuously actively transporting the fluid (20,26) through the module housing (10).

11. The assembly according to claim 9 or 10, further having a mechanical or electromagnetic drive unit (90) for rotationally driving the roller (30) in the module (10).

12. Method for inactivating pathogens in a biological fluid (20), comprising the steps of:
a) supplying the biological fluid (20) optionally containing active pathogens to the cassette according to any one of claims 1 to 8,
b) rotating the roller (30) in the module housing (10) of the cassette so that a continuous liquid film (22) of the biological liquid (20) of predeterminable thickness is formed on the rotating roller surface (32),
c) irradiating the liquid film (22) on the roller surface (32) with ionising beta radiation at a dose effective to inactivate the pathogens of the biological liquid (20),
d) collecting the irradiated liquid (26) with inactive pathogens from the roller surface (32).

13. Use of the cassette according to any one of claims 1 to 8 for continuous inactivation of pathogens in a biological fluid by means of ionising beta radiation.

## Revendications

1. Cassette pour la production d'un film liquide continu (22) à partir d'un liquide biologique fourni (20), convenant à l'irradiation du film liquide produit (22) et à l'inactivation continue des agents pathogènes dans le liquide biologique dans un dispositif d'irradiation continue à dose contrôlée,
avec un boîtier de module (10) contenant :
Bac (12) pour recevoir le liquide (20),
canal d'alimentation (14) pour alimenter le liquide (20) dans le bac (12),
rouleau cylindrique (30) qui est immergé dans le bac (12) et le liquide (20) et qui peut y tourner,
lèvre d'essuyage (40) sur le côté de sortie (36) du rouleau (30) en contact étroit avec la surface du rouleau (32) pour essuyer le film liquide (22) formé sur la surface du rouleau rotatif (32) pendant la rotation du rouleau (30), et
un canal d'évacuation (18) pour recevoir et évacuer le liquide (26) essuyé par la lèvre d'essuyage (40), et
avec un couvercle de boîtier (60) pour fermer hermétiquement le boîtier de module (10),
**caractérisé en ce que** le couvercle de boîtier (60) comporte une fenêtre métallique (62) étanche aux gaz et aux liquides qui est perméable au rayonnement bêta,
**en ce que** le boîtier de module (10) comprend un canal de débordement (16) pour évacuer le liquide en excès du bac (12) afin d'établir un niveau de liquide (24) dans le plateau (12), et
**en ce que** la cassette est stérilisable et remplaçable pour une utilisation récurrente sur l'ensemble d'irradiation à dose contrôlée continue.

2. La cassette selon la revendication 1, dans laquelle le boîtier du module (10) contenant en outre :
Un élément de formation d'espace (50) sur le côté amont (34) du rouleau (30) pour former et uniformiser le film liquide (22) sur la surface du rouleau (32), l'élément de formation d'espace (50) sur le côté amont (34) du rouleau (30) étant espacé de la surface du rouleau (32) de manière à former un espace capillaire (52), l'espace capillaire (52) s'étendant jusqu'au-dessus du niveau du liquide (24).

3. La cassette selon la revendication 2, dans laquelle l'élément de formation d'espace (50) est ajustable ou remplaçable dans son espacement par rapport à la surface du rouleau (32) pour contrôler l'épaisseur du film liquide (22) formé à cet endroit.

4. La cassette selon l'une quelconque des revendications précédentes, dans laquelle la lèvre d'essuyage (40) du côté de la sortie (36) du rouleau (30) est dirigée à l'opposé du sens de sortie du rouleau rotatif.

5. La cassette selon l'une quelconque des revendications 1 à 3, dans laquelle la lèvre d'essuyage (40) du côté de la sortie (36) du rouleau (30) est dirigée dans le sens de la sortie du rouleau rotatif.

6. La cassette selon la revendication 5, dans laquelle le canal d'évacuation (18) est formé comme au moins un tube faisant saillie dans le canal (58) formé entre la lèvre d'essuyage (40) et le côté de sortie (36) du rouleau (30).

7. La cassette selon l'une quelconque des revendications précédentes, dans laquelle le boîtier de module (10) comprend un accouplement (92) pour entraîner extérieurement la rotation du rouleau (30) par l'intermédiaire d'une unité d'entraînement accouplable (90) disposée à l'extérieur du boîtier de module (10).

8. La cassette selon la revendication 7, dans laquelle le rouleau (30) comprend des éléments magnétiques pour entraîner extérieurement la rotation du rouleau (30) par l'intermédiaire d'une unité d'entraînement mécanique ou électromagnétique (90) disposée à l'extérieur du boîtier de module (10) et pouvant être couplée magnétiquement au rouleau (30) par l'accouplement (92).

9. Dispositif pour l'irradiation continue à dose contrôlée d'un fluide biologique (20) pour l'inactivation continue des agents pathogènes dans le fluide biologique (20), comprenant :
la cassette remplaçable de l'une quelconque des revendications précédentes ; et
une source (80) de rayonnement bêta,
dans lequel la cassette est directement couplée à la source de rayonnement (80).

10. Dispositif selon la revendication 9, comprenant en outre une ou plusieurs pompes (94, 96) pour transporter activement et en continu le fluide (20, 26) à travers le boîtier de module (10).

11. Dispositif selon la revendication 9 ou 10, comprenant en outre une unité d'entraînement mécanique ou électromagnétique (90) pour entraîner en rotation le rouleau (30) dans le module (10).

12. Procédé d'inactivation d'agents pathogènes dans un fluide biologique (20), comprenant les étapes consistant à :
a) fournir le fluide biologique (20) contenant éventuellement des agents pathogènes actifs à la cassette selon l'une quelconque des revendications 1 à 8,
b) faire tourner le rouleau (30) dans le boîtier de module (10) de la cassette de sorte qu'un film liquide continu (22) du liquide biologique (20) d'une épaisseur prédéterminable se forme sur la surface du rouleau rotatif (32),
c) irradier le film liquide (22) sur la surface du rouleau (32) avec un rayonnement bêta ionisant à une dose efficace pour inactiver les agents pathogènes du liquide biologique (20),
d) recueillir le liquide irradié (26) avec des agents pathogènes inactifs sur la surface du rouleau (32).

13. Utilisation de la cassette selon l'une quelconque des revendications 1 à 8 pour l'inactivation en continu d'agents pathogènes dans un fluide biologique au moyen d'un rayonnement bêta ionisant.
